# EUROPÄISCHE PATENTANMELDUNG

(11) **EP 1 693 337 A1**
(43) Veröffentlichungstag der Anmeldung: **23.08.2006**
(21) Anmeldenummer: 05003588.0
(22) Anmeldetag: 18.02.2005
(51) Int. Cl.: B81B 1/00, C12Q 1/68, B01L 3/00

(54) **Makroporöser Träger für chemische Amplifikationsreaktionen**

(71) Anmelder: Infineon Technologies AG, 81669 München (DE)
(72) Erfinder: Dertinger, Stephan Dr., 80335 München (DE); Klühr, Macro, 80335 München (DE); Haneder, Thomas, 85221 Dachau (DE)
(74) Vertreter: Müller-Boré & Partner Patentanwälte

(57) **Zusammenfassung**

Die vorliegende Erfindung betrifft Verfahren zur Durchführung einer Amplifikation von Nukleinsäuren in Poren (2) eines flächig ausgebildeten makroporösen Trägermaterials (1), wobei ein vorbestimmter Teil des für die Amplifikation einer Nukleinsäure benötigten Reaktionsgemischs (3) in Poren des Trägermaterials bereitgestellt werden. Ferner betrifft die vorliegende Erfindung Vorrichtungen, die ein flächig ausgebildetes makroporöses Trägermaterial (1) mit einer Vielzahl von Poren (2) umfassen, wobei in den Poren ein vorbestimmter Teil eines Reaktionsgemischs (3) zur Durchführung einer Amplifikation von Nukleinsäuren bereitgestellt wird.

## Beschreibung

Die vorliegende Erfindung betrifft Verfahren zur Durchführung einer Amplifikation von Nukleinsäuren in Poren eines flächig ausgebildeten makroporösen Trägermaterials, wobei ein vorbestimmter Teil des für die Amplifikation einer Nukleinsäure benötigten Reaktionsgemischs in zwei oder mehreren Poren des Trägermaterials bereitgestellt werden. Ferner betrifft die vorliegende Erfindung Vorrichtungen, die ein flächig ausgebildetes makroporöses Trägermaterial mit einer Vielzahl von Poren umfassen, wobei in den Poren ein vorbestimmter Teil eines Reaktionsgemischs zur Durchführung einer Amplifikation von Nukleinsäuren bereitgestellt wird.

Verfahren, die eine Untersuchung von Nukleinsäuren einschließen, finden in der molekularbiologischen Forschung aber auch in der Diagnostik von zahlreichen Erkrankungen immer mehr Anwendung. Zu diesem Zweck wird die entsprechende Nukleinsäure in der Regel amplifiziert. Bei der Polymerasekettenreaktion (PCR) handelt es sich um ein in vitro Verfahren zur gezielten Amplifikation von DNA-Abschnitten. Kurze Oligonukleotide (Primer) binden spezifisch an beiden Enden des gewünschten DNA-Abschnitts und initiieren so die exponentielle Vervielfältigung durch die DNA-Polymerase. Theoretisch kann bei einer 100%igen Effizienz der Reaktion eine Vermehrung von 2ⁿ Kopien der Ausgangsmenge an Fragment-Kopien erreicht werden, wobei n die Anzahl der Amplifikationszyklen ist.

Für die Verwendung der Nukleinsäure-Amplifikation in der Grundlagenforschung, der Diagnostik und der Therapieüberwachung erhält die Automatisierung, Parallelisierung und Miniaturisierung bei gleichzeitiger Erhöhung der Spezifität eine immer größere Bedeutung. Neben der Zeit- und Kostenersparnis, die mit einer Automatisierung verbunden sind, besteht ein großer Vorteil darin, daß Unterschiede der Reaktionsbedingungen bei einzelnen Reaktionsansätzen bedingt durch Pipettierungenauigkeiten oder leichte Temperatur- oder Zeitschwankungen bei der Amplifikation oder gerätebedingte Ableseungenauigkeiten beim photometrischen Nachweis der Amplifikationsprodukte minimiert werden. Dies bedeutet, daß je mehr Bestandteile eines Reaktionsgemischs für die zu vergleichenden Reaktionsansätze in einem gemeinsamen Mastermix angesetzt werden können und je mehr Reaktionen gleichzeitig durchgeführt und analysiert werden können, desto mehr sind die Ergebnisse miteinander vergleichbar. Da es sich bei der PCR um eine exponentielle Amplifikation von Nukleinsäuren handelt, potenzieren sich geringe Unterschiede in den Reaktionsbedingungen und können sich somit auf das Ergebnis deutlich auswirken. Die Spezifität etablierter PCR-Bedingungen kann wesentlich durch eine Verkürzung der Aufheiz- und Abkühl-Intervalle der Reaktionsgemische während der Amplifikation erhöht werden, da hierdurch die Gefahr einer unspezifischen Bindung der Primer vermindert wird. Ein weiterer Faktor zur Steigerung der Effizienz der Amplifikation von Nukleinsäuren liegt in der Reduktion des Reaktionsvolumens. Je geringer das Reaktionsvolumen ist, desto schneller wird eine gleichmäßige Temperaturerhöhung bzw. -erniedrigung des gesamten Volumens erreicht. Zusätzlich können mehr Reaktionen und somit mehr Analysen mit einem Probenmaterial durchgeführt werden. Insbesondere bei begrenzt zur Verfügung stehendem Patientenmaterial, z.B. aus einer Biopsie, oder bei Probenmaterial, dessen Gewinnung mit großem Aufwand verbunden ist, z.B. cDNA, stellt dies einen großen Vorteil dar.

Die im Stand der Technik bekannten Konzepte zur Miniaturisierung und Parallelisierung der Amplifikation von Nukleinsäuren nutzen bevorzugt planare "Lab-on-a-chip" Anordnungen (Kopp et al., Science (1998) 280, 1046-1048). Diese Chip-Systeme arbeiten mit einem Durchflußkonzept, bei dem die PCR kontinuierlich mit dem Durchlaufen der Reaktionslösungen durch unterschiedliche Temperaturzonen abläuft. Die Nachteile dieses Konzepts sind neben der Tatsache, daß die Anzahl der Temperaturzyklen durch die Chipgeometrie festgelegt ist und nicht variiert werden kann, eine starke Einschränkung des Grads der Parallelisierung aufgrund der experimentellen Komplexität des PCR-Durchflußkonzepts und der hohe Platzbedarf pro Chip durch die planare Anordnung der PCR-Durchflußreaktoren, der zu hohen Herstellungskosten führt. In der Patentanmeldung US 2001/0055765 A1 wird eine Vorrichtung und ein Verfahren zur Durchführung von chemischen und biochemischen Reaktionen in offenen Reaktionskammern eines planaren Substrats offenbart, wobei eine Vielzahl von flüssigen Proben in die einzelnen Reaktionskammern gegeben wird, was enorm arbeitsaufwendig, zeitaufwendig und kostenintensiv ist. Die vorstehend genannte US-Anmeldung basiert, ebenso wie alle derzeit existierenden Konzepte für Mikro- und Nano-Titerplatten, auf dem Konzept "ein Reaktionsansatz in einem Reaktionsvolumen". Mit zunehmender Miniaturisierung steigen jedoch die Anforderungen an die Dispensiertechnologie:
(i) Es müssen immer kleinere Volumina mit hoher Genauigkeit und hoher Reproduzierbarkeit dispensiert werden. Erschwerend kommt hinzu, daß insbesondere in den Life Sciences und der molekularen Diagnostik häufig Reaktionslösungen mit stark unterschiedlichen Benetzungseigenschaften (z.B. mit Detergenzien) und Viskositäten (z.B. hoch konzentrierte Proteinlösungen oder Blut) pipettiert werden müssen, wodurch die Genauigkeit und die Reproduzierbarkeit leiden.
(ii) Die Anforderungen an die Genauigkeit und die Reproduzierbarkeit der Positionierung der abzusetzenden Lösung, um die einzelnen diskreten Reaktionsgefäße anzusteuern, steigen. Solche Präzisions-Dispensiersysteme sind aufwendig in der Wartung und teuer in der Herstellung. Besonders für das Umfeld von Service- und Routinelabors sind Präzisions-Dispensiersysteme ungeeignet.

Somit sind Verfahren unter Anwendung eines miniaturisierten Trägers mit entsprechenden Reaktionsgefäßen gewünscht, welche nur geringe Anforderungen an die Positionierungsgenauigkeit der Dispensiervorrichtung stellen und gleichzeitig eine sehr exakte und reproduzierbare Beschickung kleinster Flüssigkeitsmengen ermöglichen.

Der vorliegenden Erfindung liegt somit die Aufgabe zugrunde, neuartige Verfahren und Vorrichtungen für die verbesserte Durchführung der Amplifikation von Nukleinsäuren bereitzustellen.

Diese Aufgabe wird durch die in den Ansprüchen gekennzeichneten Ausführungsformen gelöst.

Insbesondere wird ein Verfahren zur Amplifikation von Nukleinsäuren in Poren eines flächig ausgebildeten makroporösen Trägermaterials, das gegenüberliegend eine erste und zweite Oberfläche aufweist, wobei über den gesamten Oberflächenbereich verteilt eine Vielzahl von diskreten Poren mit einem Durchmesser im Bereich von 500 nm bis 100 µm, einem Aspektverhältnis von Porentiefe zu Porenöffnung von mindestens 10:1 und einer Porendichte von 10⁴ bis 10⁸/cm² angeordnet ist, bereitgestellt, umfassend
(a) das Bereitstellen von einem vorbestimmten Teil eines zur Amplifikationsreaktion befähigten Reaktionsgemischs in mindestens einem Bereich des Trägermaterials, der mindestens zwei Poren umfaßt, derart daß der Teil des Reaktionsgemischs nicht-kovalent an die Porenwand des Trägermaterials gebunden wird, so daß gleichzeitig ein Reaktionsbereich bzw. eine Reaktionszone auf der Trägermaterialoberfläche definiert wird,
(b) das Hinzufügen einer Probe über das gesamte Trägermaterial, die den zur Komplettierung für eine Amplifikationsreaktion erforderlichen Teil des Reaktionsgemischs enthält,
(c) das Durchführen der Amplifikationsreaktion, und
(d) das Nachweisen von mindestens einem Amplifikationsprodukt.

Mit einem geordneten, porösen Substrat lassen sich die beiden vorstehend aufgeführten Anforderungen erfüllen, wenn man das Konzept "ein Reaktionsansatz in einem Reaktionsvolumen" aufgibt und einen Reaktionsansatz auf mehrere aneinandergrenzende Kapillaren bzw. Poren verteilt:
- Ein Reaktionsbereich wird nicht über die Geometrie des Trägersubstrats vorgegeben, sondern erst durch das Beschicken mit einer Reaktionslösung - oder Teilen davon - definiert (z.B. durch Verwendung von Spotting-Techniken wie sie auch für die Herstellung von Mikroarrays verwendet werden). Auf dem erfindungsgemäß vorgesehenen porösen Träger setzt sich eine Reaktionszone aus einer zusammenhängenden Gruppe von Kapillaren bzw. Poren zusammen, wobei ein justiertes Spotten nicht notwendig ist. Die Reaktionszonen müssen nicht nahtlos aneinander liegen, sondern können auch durch Zwischenräume, die mehrere Poren umfassen, getrennt sein.
- Durch die Sogwirkung der Kapillarkräfte, die beim Kontakt einer Lösung mit dem porösen Träger entsteht, wird eine Gruppe von diskreten Reaktionskapillaren gefüllt. Sind die Kapillaren bzw. Poren gefüllt, können sie keine weiteren Lösungen mehr aufnehmen. Der erfindungsgemäß vorgesehene poröse Träger ist daher ein selbstbegrenzendes System. Das Befüllen der Kapillaren funktioniert sowohl mit Sack- als auch mit Durchgangslöchern.

Eine weitere Miniaturisierung über die heute genutzten Formate hinaus ist mit vielen Vorteilen verbunden. Durch die kleinen Abmessungen des erfindungsgemäß eingesetzten Trägermaterials können Temperaturgradienten über die Probe vermieden werden. Weiterhin reduzieren sich die Reagenzienmengen, die für eine Befüllung des Reaktionsträgers nötig sind, und damit gleichzeitig die Kosten pro Test/Experiment. Besonders im Fall der chemischen Amplifikationen, bei denen schnelle Heiz- und Kühlraten gefordert sind, sind die geringen Abmessungen eines derart miniaturisierten Reaktionsträgers von Vorteil, da die zu transportierenden Wärmemengen klein sind und damit schnell in das System gepumpt oder von dort abgeführt werden können.

Unter Poren sind im Rahmen der vorliegenden Erfindungen sowohl Poren, die sich durch das Trägermaterial von der ersten zur zweiten Oberfläche erstrecken, d.h. Durchgangslöcher, als auch Poren, die an einem Ende verschlossen sind, d.h. Sacklöcher, zu verstehen. Vorzugsweise sind im Rahmen der vorliegenden Erfindung Poren vorgesehen, die sich durch das Trägermaterial von der ersten zur zweiten Oberfläche erstrecken.

Das makroporöse Trägermaterial der vorliegenden Erfindung unterliegt bezüglich der Materialwahl keiner Beschränkung, solange es Poren mit einem Porendurchmesser von Porendurchmesser von 500 nm bis 100 µm, vorzugsweise 2 bis 20 µm, und eine Porendichte von 10⁴ bis 10⁸ Poren/cm² aufweist. Insbesondere kann das erfindungsgemäß verwendete makroporöse Trägermaterial auf Basis von Kunststoff, Glas, Al₂O₃ oder Silizium sein. Vorzugsweise wird makroporöses Silizium und noch mehr bevorzugt partiell oxidiertes makroporöses Silizium als makroporöses Trägermaterial im Rahmen der vorliegenden Erfindung eingesetzt.

Das vorzugsweise verwendete makroporöse Silizium kann dabei dotiert, vorzugsweise n-dotiert, oder undotiert sein. Ein solches makroporöses Silizium kann beispielsweise nach dem in EP-A1-0 296 348 beschriebenen Verfahren hergestellt werden. Die Herstellung der Lochöffnungen bzw. Poren erfolgt bevorzugt auf elektrochemischem Wege, wobei eine elektrochemische Ätzung in einem flußsäurehaltigen Elektrolyten unter Anlegen eines konstanten oder sich zeitlich ändernden Potentials durchgeführt wird, wobei die aus Silizium bestehende Schicht oder das Substrat als positiv gepolte Elektrode einer Elektrolysierzelle geschaltet wird. Die Herstellung derartiger Löcher kann bespielsweise erreicht werden, wie in V. Lehmann, J. Electrochem. Soc. 140, 1993, Seiten 2836 ff., beschrieben. Im Rahmen der vorliegenden Erfindung können als makroporöses Substrat beispielsweise aber auch andere Halbleitersubstrate, wie z.B. GaAs-Substrate, oder mit Si₃N₄ beschichtete Glassubstrate vorgesehen werden.

In besonders bevorzugter Weise kann als makroporöses Trägermaterial im Rahmen der vorliegenden Erfindung ein partiell oxidiertes makroporöses Silizium eingesetzt werden, wie es in WO 03/089925 beschrieben ist, auf die hierin vollständig Bezug genommen wird. Mit einem solchen partiell oxidierten makroporösen Silizium ist ein flächig ausgebildetes makroporöses Trägermaterial auf Silizium-Basis gemeint, das über den gesamten Oberflächenbereich verteilt eine Vielzahl von Poren mit einem Durchmesser im Bereich von 500 nm bis 100 µm, einem Aspektverhältnis von Porentiefe zu Porenöffnung von mindestens 10:1 und einer Porendichte von 10⁴ bis 10⁸/cm² aufweist, wobei der Träger mindestens einen Bereich aufweist, der mehrere bzw. eine Vielzahl von Poren mit Porenwänden aus SiO₂ umfasst, und wobei dieser Bereich von einem zu den Längsachsen der Poren im wesentlichen parallel angeordneten, zu den Oberflächen hin offenen Rahmen aus Wänden mit einem Siliziumkern umgeben ist, wobei der Kern aus Silizium über den Querschnitt zur Außenseite der den Rahmen bildenden Wände hin in Siliziumdioxid übergeht. Ein solches partiell oxidiertes makroporöses Silizium weist somit lokal transparente Bereichen aus SiO₂ auf, wobei diese transparenten Bereiche wiederum von einem Rahmen aus Wänden mit Siliziumkern umgeben sind. Mit anderen Worten, es liegen lokal vollständig transparente Bereiche aus SiO₂ vor, die durch nicht-transparente Wände mit Siliziumkern, welche in quasi eine Sekundärstruktur bilden, voneinander getrennt sind.

Dabei kann das als makroporöses Trägermaterial im Rahmen der vorliegenden Erfindung verwendete, partiell oxidierte makroporöse Silizium auch derart gestaltet sein, wie es in DE 10 2004 018846.7 beschrieben ist, auf die hier vollständig Bezug genommen wird, nämlich dass jeder einzelne Rahmen innerhalb der Gesamtheit aller Rahmen aus Wänden mit einem Siliziumkern vollständig von den ihn umgebenden Rahmen bzw. den benachbarten Rahmen räumlich isoliert ist. Die Siliziumwände der einzelnen Bereiche bzw. Kompartments hängen dann nicht mehr zusammen bzw. berühren sich nicht, sondern sind über Bereiche mit Porenwänden aus SiO₂ voneinander vollständig getrennt. Aufgrund dieser strukturellen Anordnung wird eine räumliche Entkopplung der im Zuge der Herstellung eines solchen partiell oxidierten makroporösen Siliziums durch die Volumenverdopplung beim Übergang von Silizium zu Siliziumdioxid lokal entstehenden Spannungen erreicht. In den durchoxidierten Bereichen sind die Wände zwischen den Poren vollständig aus SiO₂ aufgebaut.

Die erfindungsgemäß vorgesehene dreidimensionale Anordnung der Amplifikationsreaktoren nutzt die Tiefe des Substrats und erlaubt eine hohe Integration von Amplifikationsreaktionszonen und damit eine kostengünstige Herstellung des Reaktionsträgers. Es wird eine vollständige Separierung der Reaktionsvolumina erreicht, so daß es zu keinen Querkontaminationen kommt. Das erfindungsgemäße makroporöse Trägermaterial weist einen Porendurchmesser von 500 nm bis 100 µm, vorzugsweise 2 bis 10 µm auf. Durch diese Minimierung der Reaktionsvolumina auf ungefähr 10 pl bis 500 nl, vorzugsweise 1,5 bis 20 nl werden die Kosten pro Amplifikationsreaktion gesenkt. Die Kapillarkräfte in den Poren erleichtern das Befüllen der Poren. Die geringe thermische Kapazität und ein guter Wärmeübergang auf das Reaktionsgemisch in der Pore ermöglicht sehr schnelle Temperaturänderungen im System und erlaubt damit die Durchführung von selektiven und sensitiven Amplifikationen in sehr kurzer Zeit mit Aufheiz- bzw. Abkühlungsraten von ungefähr 3 bis 10°C/s. Das Aufheizen und Abkühlen des Trägermaterials kann entweder durch einen externen Heiz-/Kühlblock (z.B. Heizwendel/Druckluftkühlung oder Peltierelement), vorzugsweise mit einem Temperatursensor (z.B. Pt100), oder durch ein auf dem Trägermaterial integriertes Heizelement (z.B. ein Heizwendel), vorzugsweise mit einem Temperatursensor (z.B. Pt100), erfolgen.

Die Formulierung "Amplifikation von Nukleinsäuren" schließt jede enzymatische Amplifikation einer Nukleinsäure, vorzugsweise durch ein Enzym, das eine Polymerase-Aktivität aufweist, ein. Beispiele für solche Enzyme sind die Taq Polymerase oder die Pfu Polymerase. Die Amplifikation kann durch eine Polymeraseketten-Reaktion (PCR) durchgeführt werden.

Beispiele für eine erfindungsgemäße Amplifikation von Nukleinsäuren sind die Echtzeit-PCR, die eingebettete (nested) PCR, die asymetrische PCR, die Gradienten-PCR, die RT-PCR und die Methylierungs-spezifische PCR.

Die Echtzeit-Polymerasekettenreaktion (Echtzeit-PCR, real-time PCR) vervielfältigt DNA-Fragmente in einer PCR und erlaubt zudem eine kontinuierliche Beobachtung der DNA-Amplifikation durch das Messen der Fluoreszenzsignale. Dies ist sowohl durch das Verwenden von Farbstoffen, die unspezifisch an doppelsträngige Nukleinsäuren binden, möglich als auch durch den Einsatz Sequenz-spezifischer Sonden, die an Fluoreszenzfarbstoffe gebunden sind.

Bei der eingebetteten (nested) PCR wird zunächst ein Nukleinsäure-Fragment mittels PCR amplifiziert. Dieses PCR-Produkt dient in einer zweiten PCR als Ziel-Nukleinsäure. Mit Hilfe eines zweiten Primerpaars, das innerhalb des ersten PCR-Fragments hybridisiert, wird ein kleineres Fragment in einer anschließenden PCR amplifiziert.

Die zu amplifizierende Nukleinsäure kann jede natürlich vorkommende oder künstlich hergestellte Nukleinsäure sein. Dies schließt sowohl doppel- und einzelsträngige DNA- oder RNA-Moleküle als auch Nukleinsäuren mit modifizierten Basen ein. Ferner schließt dies auch Nukleinsäuren ein, die vor der Amplifikation modifiziert wurden, beispielsweise durch die Behandlung mit Natriumbisulfit zur Umwandlung von methyliertem Cytosin zu Uracil zum Nachweis methylierter DNA in einer Methylierungs-spezifischen PCR. Ferner ist auch die Amplifikation von DNA-Molekülen, die zuvor aus RNA revers transkribiert wurden, mit eingeschlossen.

Ein vorbestimmter Teil eines Reaktionsgemischs kann eine oder mehrere Verbindungen des für eine Amplifikation einer Nukleinsäure benötigten Reaktionsgemischs einschließlich Enzyme, Nukleotide (z.B. Desoxynukleotide wie dATP, dCTP, dGTP, dTTP, dUTP, oder Didesoxynukleotide wie ddATP oder ddCTP), Salze (z.B. MgCl₂), Primer (einschließlich während der Amplifikation verlängerbarer und nicht-verlängerbarer Primer), Nukleinsäuren (z.B. DNA, RNA), PNA (Peptide Nucleic Acid), Farbstoffe und Sonden sein.

Die vorbestimmten Teile bzw. deren chemische Verbindungen, wie vorstehend erläutert, eines zur Amplifikationsreaktion befähigten Reaktionsgemischs werden üblicherweise durch Eintrocknen einer diese Teile enthaltenen wässrigen Lösung an die Porenwand des Trägermaterials gebunden.

In einer bevorzugten Ausführungsform der vorliegenden Erfindung umfassen die in den Poren in Schritt (a) bereitgestellten vorbestimmten Teile Nukleotide, mindestens eine Sonde und/oder mindestens einen Farbstoff, Salze und Primer, in einer bevorzugteren Ausführungsform zusätzlich PNA, so daß zur Durchführung der enzymatischen Reaktion in Schritt (c) das zu testende Probenmaterial und das Enzym hinzugegeben werden. Auf diese Weise können, in einer besonders bevorzugten Ausführungsform, standardisierte, für einen bestimmten Nachweis bestimmte makroskopische Träger hergestellt und und an potentielle Verwender vertrieben werden. Der Verwender stellt die Probe, in welcher der Nachweis erfolgen soll, und vorzugsweise das Enzym bereit und führt die Amplifikation einer Nukleinsäure durch.

Der Begriff "Salze" umfaßt sämtliche bekannte Salze in einer Konzentration, in der sie die enzymatische Amplifikation einer Nukleinsäure nicht verhindern.

Der Begriff "Primer" umfaßt jegliche Oligonukleotide, die über Basenpaarung mit einer komplementären Nukleinsäuresequenz hybridisieren und als Startpunkt für das Hinzufügen von Nukleotiden dienen. Die Primer der vorliegenden Erfindungen können eine nachweisbare Markierung tragen, die es erlaubt, die Produkte der Nukleinsäure-Amplifikationsreaktion nachzuweisen.

Bei PNA handelt es sich um Oligonukleotide, deren Basen nicht durch eine Zucker-Phosphat-Brücke miteinander verbunden sind, sondern durch eine Amino-ähnliche Brücke. Diese Konstrukte hybridisieren Sequenz-spezifisch über Basenpaarung mit einer sehr hohen Bindungsenergie mit komplementärer Einzelstrang-DNA. Durch die gebundenen PNA ist die Polymerase nicht in der Lage, die Nukleinsäure zu amplifizieren. Sobald es zu einer Fehlpaarung zwischen PNA und Nukleinsäure kommt, sinkt die Bindungsenergie sehr stark und die PNA binden während der Amplifikationsreaktion nicht an die Nukleinsäure.

Der Begriff "Farbstoffe" bezeichnet sämtliche bekannte Farbstoffe, die zum Nachweisen von amplifizierten Nukleinsäuren verwendet werden können, einschließlich spezifisch an doppelsträngige Nukleinsäuren bindende (z.B. interkalierende) Fluoreszenzfarbstoffe wie Sybr Green oder Ethidiumbromid.

Der Begriff "Sonde" umfaßt jede bekannte Nukleotidsequenz, die für den Nachweis einer amplifizierten Nukleinsäure durch spezifische Hybridisierung verwendet werden kann, einschließlich Sonden, die mit einer nachweisbaren Markierung verknüpft sind. Eine solche nachweisbare Markierung kann beispielsweise ein Fluoreszenzfarbstoff oder eine Verbindung mit einer hohen Affinität für eine nachweisbare Markierung sein. Das in der vorliegenden Erfindung verwendete Sondensystem kann jedes bekannte Sondensystem, das für den Nachweis von PCR-Produkten verwendet werden kann, sein wie zum Beispiel TaqMan-Sonden, Molecular Beacons, Scorpion-Sonden, Light Cycler-Sonden oder andere Fluoreszenz-Resonanz-Energie-Transfer (FRET)-Sonden. TaqMan-Sonden enthalten einen "Reporter" an einem Ende und einen "Quencher", der das Emittieren eines nachweisbaren Fluoreszenzsignal durch den Reporter verhindert, am anderen Ende. Nach der Hybridisierung der Sonde mit einer komplementären Nukleotidsequenz spaltet die 5'→3'-Exonukleaseaktivität der Polymerase die Sonde, so daß der Quencher sich nicht mehr in der Nähe des Reporters befindet und ein nachweisbares Fluoreszenzsignal emittiert wird. Molecular Beacons weisen auch einen "Reporter" an einem Ende und einen "Quencher", der das Emittieren eines nachweisbaren Fluoreszenzsignal durch den Reporter verhindert, am anderen Ende auf. Eine selbst-komplementäre Sequenz an beiden Enden der Sonde führt zu einer Haarnadel-Struktur der Sonde, die aufgelöst wird, sobald die Sonde mit einer komplementären Nukleotidsequenz hybridisiert. Dieses Auflösen der Haarnadel-Struktur führt zu einer räumlichen Distanz zwischen Reporter und Quencher, die für das Emittieren eines nachweisbaren Fluoreszenzsignals durch den Reporter ausreicht. Das Fluoreszenz Resonanz Energie Transfer (FRET)-Prinzip, auf dem beispielsweise Light Cycler-Sonden basieren, beruht auf zwei Sonden, die komplementär zu benachbarten Sequenzen der Ziel-Nukleinsäure sind und mit verschiedenen Fluoreszenzfarbstoffen markiert sind. Nach der Hybridisierung der beiden Sonden mit der Ziel-Nukleinsäure wird der Farbstoff der einen Sonde angeregt und diese Anregung induziert die Emission eines nachweisbaren Fluoreszenzsignals durch den an die zweite Sonde gebundenen Farbstoff.

Der Begriff "Probe" bzw. "Probenmaterial" umfaßt jede Zusammensetzung, die eine amplifizierbare Nukleinsäure enthält. Dies schließt artifiziell synthetisierte Nukleinsäuren und isolierte Nukleinsäuren aus Viren, wie z.B. HIV, Prokaryonten, wie z.B. *Escherichia coli,* oder Eukaryonten, wie z.B. Säugetiere, mit ein. In einer bevorzugten Ausführungsform der vorliegenden Erfindung enthält das Probenmaterial DNA und/oder RNA, die aus einer vom Menschen stammenden biologischen Flüssigkeit, beispielsweise Blut, Speichel oder Urin, isoliert wurden. In einer weiteren bevorzugten Ausführungsform der vorliegenden Erfindung enthält das Probenmaterial DNA und/oder RNA, die aus einer vom Menschen stammenden Gewebe- oder Tumorprobe isoliert wurden.

In einer Ausführungsform der vorliegenden Erfindung enthält das Probenmaterial Nukleinsäuren, die für die Amplifikation gemäß der vorliegenden Erfindung vorbereitet wurden. Beispielsweise kann eine Voramplifikation des gewünschten Nukleinsäureabschnitts stattgefunden haben, z.B. in Form einer nested PCR, oder die im Probenmaterial vorhandene DNA kann durch reverse Transkription einer RNA hergestellt worden sein, z.B. in Form einer RT-PCR. Zudem kann bei der im Probenmaterial vorhandenen Nukleinsäure durch eine vorherige Behandlung mit Natriumbisulfit methyliertes Cytosin in Uracil umgewandelt worden sein, z.B. für eine Methylierungs-spezifische PCR. In einem weiteren Beispiel kann die im Probenmaterial vorhandene Nukleinsäure zuvor z.B. durch den Eisatz von Sonden oder durch eine chromatographische, elektrophoretische oder zentrifugale Auftrennung selektiv angereichert worden sein.

Das vorstehende Verfahren kann beispielsweise zum quantitativen Nachweis von Nukleinsäuren genutzt werden. In einer bevorzugten Ausführungsform werden in Schritt (a) Nukleotide, Salze, mindestens eine Sonde und/oder mindestens ein Farbstoff, Primer und gegebenenfalls PNA zur selektiven Amplifikation eines bestimmten Nukleinsäureabschnitts vorgelegt. In Schritt (b) kann Probenmaterial zugeführt werden, das so verdünnt ist, daß statistisch in eine Pore des Trägermaterials eine Kopie des zu amplifizierenden Nukleinsäureabschnitts gelangt. Wenn beispielsweise das Reaktionsgemisch PNA enthält, welche die Amplifikation einer bestimmten Variante des Nukleinsäureabschnitts, z.B. den Wildtyp, unterdrücken, kann die Häufigkeit der veränderten Variante, z.B. mutierter Nukleinsäure, detektiert werden. In einem anderen Beispiel kann das Probenmaterial so verdünnt werden, daß statistisch ein Genomäquivalent in eine Pore des Trägermaterials gelangt. Auf diese Weise können Chromosomenaberrationen (z.B. Deletionen oder Duplikationen) nachgewiesen werden. Die vorstehenden Beispiele können ihre Anwendung in der Diagnose von Gendefekten, Tumorneuerkrankungen und in der Klassifizierung von Tumoren finden. Bei den vorstehend aufgeführten Beispielen erlaubt die hohe Anzahl von Reaktionen, die bedingt durch die hohe Porenzahl des verwendeten Trägermaterials ohne zusätzliche Arbeitsschritte durchgeführt werden können, eine statistisch aussagekräftige Auswertung. Beispielsweise können sich auf einem Trägermaterial mit einer Fläche von 1 mm² je nach Abstand der Poren zueinander 10² bis 10⁶ Poren befinden. Ein weiterer Vorteil des erfindungsgemäßen Verfahrens liegt in dem Bereitstellen von im wesentlichen gleichen Bedingungen für alle parallel durchgeführten Reaktionen durch das Verwenden desselben Reaktionsgemischs und desselben Probenmaterials unter gleichen Amplifikations- und Nachweisbedingungen, so daß individuelle Schwankungen zwischen den einzelnen Reaktionsansätzen minimiert werden.

In einer besonders bevorzugten Ausführungsform dieses Verfahrens wird in Schritt (a) neben den Primern und mindestens einer Sonde und/oder mindestens eines Farbstoff für die Amplifikation bzw. Detektion des gewünschten Nukleinsäureabschnitts mindestens ein weiteres Primerpaar und mindestens eine für die zwischen diesen Primern liegende Nukleinsäuresequenz liegende spezifische Sonde vorgelegt. Das Nachweisen des Amplifikationsprodukts des zweiten Primerpaars mit Hilfe spezifischer Sonden kann als Kontrolle für die Effizienz der Amplifikationsreaktion dienen. Die Effizienz der Amplifikationsreaktion kann auch durch das Verwenden eines unspezifischen, das doppelsträngige Amplifikationsprodukt bindenden Farbstoffs kontrolliert werden.

In einer Ausführungsform umfaßt das erfindungsgemäße Verfahren weiter das Bereitstellen von mindestens einem weiteren Teil eines zur Amplifikationsreaktion befähigten Reaktionsgemischs, dessen Zusammensetzung sich von der Zusammensetzung des Teils des Reaktionsgemischs in Schritt (a) durch mindestens eine Verbindung oder durch die Konzentration mindestens einer Verbindung unterscheidet, in mindestens einem weiteren (zweiten) Bereich des Trägermaterials, der mindestens zwei Poren umfaßt und sich von dem (ersten) Bereich in Schritt (a) unterscheidet. Vorzugsweise unterscheidet sich die Zusammensetzung des mindestens einen weiteren Teils eines zur Amplifikationsreaktion befähigten Reaktionsgemischs von der Zusammensetzung des Teils des Reaktionsgemischs in Schritt (a) durch mindestens eine Verbindung.

Diese Ausführungsform betrifft somit ein Verfahren zur Amplifikation von Nukleinsäuren in Poren eines flächig ausgebildeten makroporösen Trägermaterials, das gegenüberliegend eine erste und zweite Oberfläche aufweist, wobei über den gesamten Oberflächenbereich verteilt eine Vielzahl von diskreten Poren mit einem Durchmesser im Bereich von 500 nm bis 100 µm, einem Aspektverhältnis von Porentiefe zu Porenöffnung von mindestens 10:1 und einer Porendichte von 10⁴ bis 10⁸/cm² angeordnet ist, umfassend
(a) das Bereitstellen von einem vorbestimmten Teil eines zur Amplifikationsreaktion befähigten Reaktionsgemischs in mindestens einem ersten Bereich des Trägermaterials, der mindestens zwei Poren umfaßt, derart daß der Teil des Reaktionsgemischs nicht-kovalent an die Porenwand des Trägermaterials gebunden wird, so daß gleichzeitig ein Reaktionsbereich bzw. eine Reaktionszone auf der Trägermaterialoberfläche definiert wird,
(b) das Bereitstellen von mindestens einem Teil eines Reaktionsgemischs, dessen Zusammensetzung sich von der Zusammensetzung des Reaktionsgemischs in Schritt (a) durch mindestens eine Verbindung oder durch die Konzentration mindestens einer Verbindung unterscheidet, in mindestens einem weiteren zweiten Bereich des Trägermaterials, der mindestens zwei Poren umfaßt und sich von dem ersten Bereich in Schritt (a) unterscheidet, derart daß der Teil des Reaktionsgemischs nicht-kovalent an die Porenwand des Trägermaterials gebunden wird,
(c) das Hinzufügen einer Probe über das gesamte Trägermaterial, die den zur Komplettierung für eine Amplifikationsreaktion erforderlichen Teil der Reaktionsgemische von Schritt (a) und (b) enthält,
(d) das Durchführen der Amplifikationsreaktion, und
(e) das Nachweisen von mindestens einem Amplifikationsprodukt.

Die einzelnen Bereiche können auf dem porösen Trägermaterial beliebig definiert werden.

Eine Ausführungsform des vorstehenden erfindungsgemäßen Verfahrens ist ein Nachweisverfahren, bei dem die Anwesenheit einer bestimmten Nukleinsäure in einem Probenmaterial überprüft werden soll. Insofern bei entsprechender Einteilung der Bereiche des makroporösen Trägers durch das Bereitstellen verschiedener Primer in den Schritten (a) und (b) nur durch das Variieren der Primer eine Vielzahl von Nachweisen auf einem Trägermaterial durchgeführt werden kann, kann eine Vielzahl bekannter relevanter Biomarker, z.B. alle bekannten Tumormarker oder Marker für Erkrankungen, die durch Gendefekte verursacht werden, oder eine Vielzahl bekannter Genexpressionsmarker für Tumorerkrankungen und/oder Stoffwechselerkrankungen in einem Probenmaterial in einem Arbeitsschritt getestet werden.

Ein Beispiel für einen solchen Nachweis ist das Bereitstellen von Nukleotiden, Salzen, mindestens einer Sonde und/oder mindestens eines Farbstoffs und Primern, die spezifisch an die DNA bestimmter Krankheitserreger, beispielsweise verschiedener HIV-Subtypen oder verschiedener HPV-Typen, binden, auf dem erfindungsgemäß verwendeten porösen Trägermaterial und die Zugabe von isolierter DNA aus Blutbestandteilen und einem Enzym zur Amplifikation der DNA beispielsweise zu diagnostischen Zwecken. Ein weiteres Beispiel für einen solchen Nachweis ist das Bereitstellen von Nukleotiden, Salzen, mindestens einer Sonde und/oder mindestens eines Farbstoffs und Primern, die spezifisch an die mRNA bestimmter Gene, deren Expression bei Vorliegen einer bestimmten Erkrankung hoch- oder herunterreguliert ist, auf dem erfindungsgemäß verwendeten porösen Trägermaterial und die Zugabe von isolierter RNA aus Gewebe oder Blutbestandteilen und einem Enzym zur reversen Transkription der mRNA zu DNA und anschließender Amplifikation der DNA zu diagnostischen Zwecken. Ein weiteres Beispiel für einen solchen Nachweis ist der Nachweis veränderter DNA beispielsweise zur Diagnose von Tumorerkrankungen, wenn die Veränderung der DNA, die mit Hilfe des erfindungsgemäßen Verfahrens nachgewiesen wird, für die Tumorerkrankung typisch ist. In diesem Fall können neben Nukleotiden, Salzen, Sonden und/oder Farbstoffen und Primern auch PNA, welche die Amplifikation unveränderter DNA unterdrücken, auf dem erfindungsgemäß verwendeten porösen Träger bereitgestellt werden. Bei der Zugabe des Probenmaterials und des entsprechenden Enzyms erhält der Verwender ein positives Fluoreszenzsignal, wenn in der Probe veränderte DNA vorliegt.

In einer weiteren bevorzugten Ausführungsform des erfindungsgemäßen Verfahrens werden in verschiedenen Bereichen des Trägermaterials Nukleotide, Salze, mindestens eine Sonde und/oder mindestens ein Farbstoff und verschiedene, Bereichsspezifische Nukleinsäuren bereitgestellt. Anschließend werden die restlichen Teile des Reaktionsgemisch einschließlich der, für alle Bereiche gleiche Primer hinzugefügt, die Amplifikationsreaktion durchgeführt und die Amplifikationsprodukte nachgewiesen. Diese Ausführungsform kann beispielsweise verwendet werden, wenn die cDNAs verschiedener Gewebe oder Tumoren auf dem Trägermaterial bereitgestellt werden und mit Hilfe der zugefügten Primer festgestellt werden soll, in welchen der Gewebe oder Tumore die Nukleinsäure, die mit Hilfe der Primer amplifiziert wird, exprimiert wird. Auf diese Weise kann die Expression bekannter und unbekannter Gene in verschiedenen Geweben, beispielsweise Gewebeproben verschiedener Krankheitsstadien oder Entwicklungsstufen, oder Tumoren, beispielsweise unterschiedliche Tumortypen oder unterschiedliche Tumorstadien, untersucht werden.

Üblicherweise wird das Trägermaterial vor Schritt a) einseitig verschlossen. Ferner erfolgt vor dem Durchführen der Amplifikationsreaktion in der Regel ein weiteres Verschließen auf der noch offenen Seite, d.h. ein beidseitiges Verschließen. Dadurch kann beispielsweise ein Verdunsten des Reaktionsgemischs beim Erhitzen während der Amplifikationsreaktion vermieden werden. Das Verschließen kann auf jede bekannte Art und Weise, welche die Amplifikationsreaktion nicht wesentlich beeinträchtigt, erfolgen. Beispielsweise können die Ober- bzw. die Unterseite oder beide Seiten des porösen Trägermaterials mit einer Polymer-Folie oder einer Matte auf Elastomer-Basis (z.B. Silikon) als Deckel oder Dichtung verschlossen werden oder die Poren können mit einem Mineralöl bedeckt werden. Ein weiteres Beispiel für das Verschließen des Trägermaterials ist das Verschließen der Poren mit Wachs, mikrokristallinem Wachs, Polyethylenwachs oder schnell aushärtenden Harzen, die keine Lösungsmittel enthalten oder andere chemische Komponenten abgeben, welche die Amplifikationsreaktion beeinträchtigen können.

In einer Ausführungsform des erfindungsgemäßen Verfahrens können die Poren auf mindestens einer Seite des makroporösen Trägermaterials auch unverschlossen bleiben. In diesem Fall erfolgt das Durchführen der Amplifikationsreaktion in einer Reaktionskammer, die mit Wasserdampf gesättigt ist und deren Abdeckung beheizbar ist.

Das Durchführen der Amplifikationsreaktion erfolgt durch das Bereitstellen von Bedingungen, unter denen das in dem Reaktionsgemisch enthaltene Enzym die Amplifikation einer Nukleinsäure vornimmt, bevorzugt durch das zyklische Erhitzen des Trägermaterials.

Das Nachweisen des Amplifikationsprodukts erfolgt bevorzugt photometrisch. Das Amplifikationsprodukt kann beispielsweise durch das Verwenden von spezifisch an doppelsträngige Nukleinsäuren bindende Farbstoffe oder durch das Verwenden markierter Sequenz-spezifischer Sonden nachgewiesen werden.

In einer bevorzugten Ausführungsform der vorliegenden Erfindung werden die vorbestimmten Teile bzw. deren chemische Verbindungen des Reaktionsgemischs, die in den Poren bereitgestellt werden, in einer Flüssigkeit gelöst, in die Poren gebracht (beispielsweise unter Ausnutzung der Kapillarkräfte der Poren) und anschließend wird die Flüssigkeit verdampft.

In einer besonders bevorzugten Ausführungsform des erfindungsgemäßen Verfahrens werden die bereitgestellten Teile eines zur Amplifikationsreaktion befähigten Reaktionsgemischs durch Eintrocknen bzw. Dehydratisierung an die Porenwand des Trägermaterials aufgebracht.

Die bereitgestellten Teile des zur Amplifikationsreaktion befähigten Reaktionsgemischs können in den jeweils gewünschten Bereichen des Trägermaterials durch im Stand der Technik bekannte Spotting-Verfahren aufgetragen werden.

In einer bevorzugten Ausführungsform der vorliegenden Erfindung erfolgt das Hinzufügen einer Probe über das gesamte Trägermaterial in einem Arbeitsschritt. Die Befüllung der einzelnen Poren des Trägermaterials erfolgt bevorzugt Kapillargetrieben. Die Kapillarkräfte der Poren bewirken eine definierte, gleichmäßige Befüllung der Pore, so daß ein selbstdosierendes System vorliegt.

Ferner wird eine Vorrichtung bereitgestellt, die ein flächig ausgebildetes makroporöses Trägermaterial umfaßt, das gegenüberliegend eine erste und zweite Oberfläche aufweist, wobei über den gesamten Oberflächenbereich verteilt eine Vielzahl von diskreten Poren mit einem Durchmesser im Bereich von 500 nm bis 100 µm, einem Aspektverhältnis von Porentiefe zu Porenöffnung von mindestens 10:1 und einer Porendichte von 10⁴ bis 10⁸/cm² angeordnet ist, wobei die Vorrichtung mindestens einen Bereich aufweist, in dem ein vorbestimmter Teil eines Reaktionsgemischs zur Durchführung einer Amplifikation einer Nukleinsäure in mindestens zwei Poren nicht-kovalent gebunden ist.

Weiterhin wird eine Vorrichtung bereitgestellt, die ein flächig ausgebildetes makroporöses Trägermaterial umfaßt, das gegenüberliegend eine erste und zweite Oberfläche aufweist, wobei über den gesamten Oberflächenbereich verteilt eine Vielzahl von diskreten Poren mit einem Durchmesser im Bereich von 500 nm bis 100 µm, einem Aspektverhältnis von Porentiefe zu Porenöffnung von mindestens 10:1 und einer Porendichte von 10⁴ bis 10⁸/cm² angeordnet ist, wobei die Vorrichtung mindestens einen ersten Bereich aufweist, in dem sich ein vorbestimmter Teil eines Reaktionsgemischs zur Durchführung einer Amplifikation von Nukleinsäuren in mindestens zwei Poren nicht-kovalent gebunden befindet, und einen zweiten Bereich aufweist, in dem sich ein vorbestimmter Teil eines Reaktionsgemischs zur Durchführung einer Amplifikation von Nukleinsäuren, der sich von dem ersten Teil durch mindestens eine Verbindung unterscheidet, in mindestens zwei Poren nicht-kovalent gebunden befindet.

Mit "nicht kovalent gebunden" wird im Rahmen der vorliegenden Erfindung das Aufbringen bzw. Anordnen von chemischen Verbindungen, welche den vorbestimmten Teil eines zur Amplifikationsreaktion befähigten Reaktionsgemischs bilden, wie vorstehend ausgeführt, in bzw. auf den jeweiligen Porenwänden des makroporösen Trägermaterials, so daß die Verbindungen an diesen Wänden fest anhaften, ohne mit diesen jedoch durch chemische kovalente Bindungen verknüpft zu sein, verstanden. Dieses feste Anhaften bzw. Physisorption kann durch entsprechendes Eintrocknen einer diese vorbestimmten Teile bzw. die diesbezüglichen Verbindungen enthaltenen wässrigen Lösung erfolgen. Die Verbindungen haften dabei reversibel, unzerstörbar und quantitativ ablösbar an den Porenwänden des makroporösen Trägermaterials.

In den erfindungsgemäßen Verfahren bzw. in den erfindungsgemäßen Vorrichtungen ist über den gesamten Oberflächenbereich des flächig ausgebildeten makroporösen Trägermaterials verteilt eine Vielzahl von üblicherweise periodisch angeordneten Poren, welche sich von einer Oberfläche zur gegenüberliegenden Oberfläche des Trägermaterials erstrecken, angeordnet. Im Rahmen der vorliegenden Erfindung können auf dem flächig ausgebildeten makroporösen Trägermaterial bereichsweise auch Sacklöcher, d.h. nur nach einer der Oberflächenseiten geöffnete Poren, vorgesehen werden.

Das in den erfindungsgemäßen Verfahren bzw. in den erfindungsgemäßen Vorrichtungen eingesetzte makroporöse Trägermaterial weist einen Porendurchmesser von 500 nm bis 100 µm, vorzugsweise 2 bis 20 µm auf. Hieraus ergeben sich Aspektverhältnisse von Porentiefe zu Porenöffnung von größer 10:1, vorzugsweise von größer 40:1. Die Dicke des makroporösen Trägermaterials beträgt üblicherweise 100 bis 5.000 µm, vorzugsweise 300 bis 600 µm. Der Abstand von Porenmitte zu Porenmitte (Pitch), d.h. zweier zueinander benachbarter bzw. angrenzender Poren beträgt üblicherweise 1 bis 100 µm, vorzugsweise 3 bis 50 µm. Die Porendichte liegt üblicherweise im Bereich von 10⁴ bis 10⁸/cm².

Das makroporöse Trägermaterial der vorliegenden Erfindung unterliegt bezüglich der Materialwahl keiner Beschränkung, solange es Poren mit einem Porendurchmesser von Porendurchmesser von 500 nm bis 100 µm, vorzugsweise 2 bis 10 µm aufweist. Insbesondere kann das erfindungsgemäß verwendete makroporöse Trägermaterial auf Basis von Glas, Al₂O₃ oder Silizium sein. Vorzugsweise wird makroporöses Silizium und noch mehr bevorzugt partiell oxidiertes makroporöses Silizium als makroporöses Trägermaterial im Rahmen der vorliegenden Erfindung eingesetzt.

Das vorzugsweise verwendete makroporöse Silizium kann dabei dotiert, vorzugsweise n-dotiert, oder undotiert sein. Ein solches makroporöses Silizium kann beispielsweise nach dem in EP-A1-0 296 348 beschriebenen Verfahren hergestellt werden. Die Herstellung der Lochöffnungen bzw. Poren erfolgt bevorzugt auf elektrochemischem Wege, wobei eine elektrochemische Ätzung in einem flußsäurehaltigen Elektrolyten unter Anlegen eines konstanten oder sich zeitlich ändernden Potentials durchgeführt wird, wobei die aus Silizium bestehende Schicht oder das Substrat als positiv gepolte Elektrode einer Elektrolysierzelle geschaltet wird. Die Herstellung derartiger Löcher kann bespielsweise erreicht werden, wie in V. Lehmann, J. Electrochem. Soc. 140, 1993, Seiten 2836 ff., beschrieben. Im Rahmen der vorliegenden Erfindung können als makroporöses Substrat beispielsweise aber auch andere Halbleitersubstrate, wie z.B. GaAs-Substrate, oder mit Si₃N₄ beschichtete Glassubstrate vorgesehen werden.

In besonders bevorzugter Weise kann als makroporöses Trägermaterial im Rahmen der vorliegenden Erfindung ein partiell oxidiertes makroporöses Silizium eingesetzt werden, wie es in WO 03/089925 beschrieben ist, auf die hierin vollständig Bezug genommen wird. Mit einem solchen partiell oxidierten makroporösen Silizium ist ein flächig ausgebildetes makroporöses Trägermaterial auf Silizium-Basis gemeint, das über den gesamten Oberflächenbereich verteilt eine Vielzahl von Poren mit einem Durchmesser im Bereich von 500 nm bis 100 µm aufweist, wobei der Träger mindestens einen Bereich aufweist, der mehrere Poren mit Porenwänden aus SiO₂ umfasst, und wobei dieser Bereich von einem zu den Längsachsen der Poren im wesentlichen parallel angeordneten, zu den Oberflächen hin offenen Rahmen aus Wänden mit einem Siliziumkern umgeben ist, wobei der Kern aus Silizium über den Querschnitt zur Außenseite der den Rahmen bildenden Wände hin in Siliziumdioxid übergeht. Ein solches partiell oxidiertes makroporöses Silizium weist somit lokal transparente Bereichen aus SiO₂ auf, wobei diese transparenten Bereiche wiederum von einem Rahmen aus Wänden mit Siliziumkern umgeben sind. Mit anderen Worten, es liegen lokal vollständig transparente Bereiche aus Si0₂ vor, die durch nicht-transparente Wände mit Siliziumkern, welche in quasi eine Sekundärstruktur bilden, voneinander getrennt sind.

Dabei kann das als makroporöses Trägermaterial im Rahmen der vorliegenden Erfindung verwendete, partiell oxidierte makroporöse Silizium auch derart gestaltet sein, wie es in DE 10 2004 018846.7 beschrieben ist, auf die hier vollständig Bezug genommen wird, nämlich dass jeder einzelne Rahmen innerhalb der Gesamtheit aller Rahmen aus Wänden mit einem Siliziumkern vollständig von den ihn umgebenden Rahmen bzw. den benachbarten Rahmen räumlich isoliert ist. Die Siliziumwände der einzelnen Bereiche bzw. Kompartments hängen dann nicht mehr zusammen bzw. berühren sich nicht, sondern sind über Bereiche mit Porenwänden aus SiO₂ voneinander vollständig getrennt. Aufgrund dieser strukturellen Anordnung wird eine räumliche Entkopplung der im Zuge der Herstellung eines solchen partiell oxidierten makroporösen Siliziums durch die Volumenverdopplung beim Übergang von Silizium zu Siliziumdioxid lokal entstehenden Spannungen erreicht. In den durchoxidierten Bereichen sind die Wände zwischen den Poren vollständig aus SiO₂ aufgebaut.

Die Poren in den erfindungsgemäßen Verfahren bzw. in den erfindungsgemäßen Vorrichtungen können beispielsweise im wesentlichen rund oder ellipsenförmig gestaltet sein. Wird als makroporöses Trägermaterial im Rahmen der vorliegenden Erfindung insbesondere ein partiell oxidiertes makroporöses Silizium eingesetzt, so können die Poren mit Porenwänden aus Si0₂ auch im wesentlichen quadratisch ausgebildet sein. Der Rahmen aus Wänden mit einem Siliziumkern kann dann in im wesentlichen quadratischer oder rechteckiger Form vorliegen.

Des weiteren wird die Verwendung eines wie vorstehend beschriebenen flächig ausgebildeten makroporösen Trägermaterials für die Amplifikation von Nukleinsäuren bereitgestellt.

Die vorliegende Erfindung wird nachfolgend unter Bezugnahme auf die Figuren beispielhaft beschrieben. Es zeigt:
- Fig. 1: Querschnittsansichten (A)bis (D) verschiedener Ausführungsformen der erfindungsgemäßen Vorrichtung,
- Fig. 2: eine schematische Draufsicht einer Ausführungsform einer erfindungsgemäßen Vorrichtung,
- Fig. 3: Querschnittsansichten (A), (B) und (C) verschiedener Ausführungsformen der Durchführung der Amplifikationsreaktion und
- Fig. 4: Querschnittsansichten (A), (B) und (C) verschiedener Stadien des Hinzufügens einer Probe über das gesamte Trägermaterial.
- Fig. 5: Querschnittsansichten (A), (B), (C), (D) und (E) verschiedener Stadien einer Ausführungsform des Bereitstellens des zur Amplifikationsreaktion befähigten Reaktionsgemischs auf dem Trägermaterial.
- Fig. 6: Querschnittsansichten (A), (B), (C), (D) und (E) verschiedener Stadien einer weiteren bevorzugten Ausführungsform des Bereitstellens des zur Amplifikationsreaktion befähigten Reaktionsgemischs auf dem Trägermaterial.

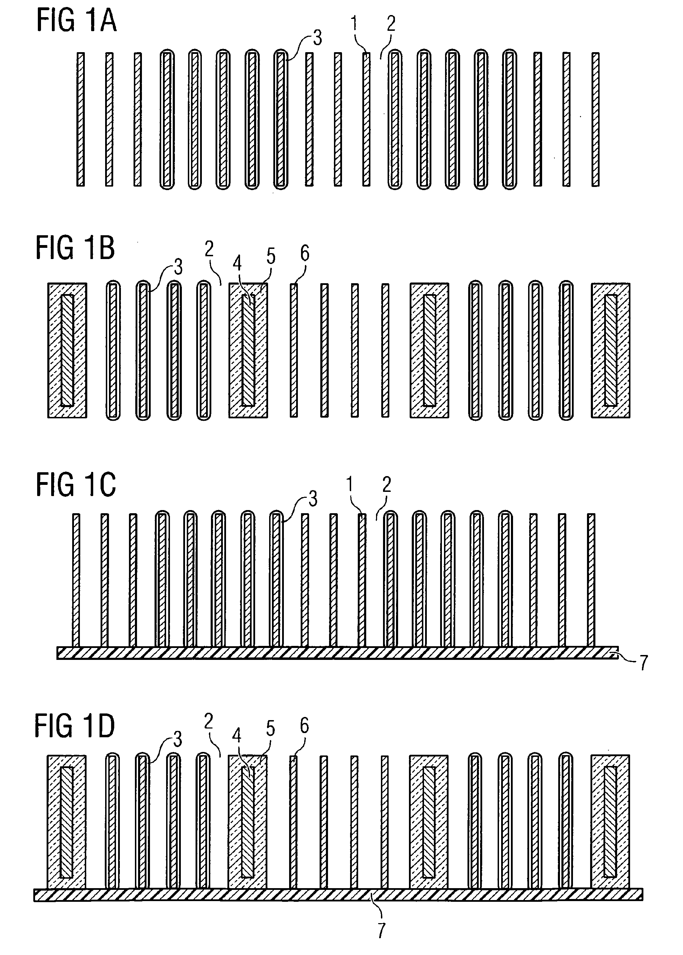

- Fig. 1: zeigt eine Querschnittsansicht (A) einer Ausführungsform
einer erfindungsgemäßen Vorrichtung auf Basis von makroporösem Trägermaterial wie beispielsweise Glas, Al₂O₃ oder Silizium, worin (1) eine Porenwand, (2) eine Pore und (3) eingetrocknete Verbindungen, den vorbestimmten Teil eines zur Amplifikationsreaktion befähigten Reaktionsgemischs bildend, darstellen; (B) eine Querschnittsansicht einer Ausführungsform einer erfindungsgemäßen Vorrichtung auf Basis von partiell oxidiertem makroporösen Silizium, worin (3) eingetrocknete Verbindungen, den vorbestimmten Teil eines zur Amplifikationsreaktion befähigten Reaktionsgemischs bildend, (4) den Siliziumkern einer Porenwand, (5) SiO₂ und (6) eine Porenwand aus SiO₂ darstellen; eine Querschnittsansicht der in (A) gezeigten Ausführungsform mit einem an der unteren Oberflächenseite angeordneten Deckel (7) auf Basis von z.B. Elastomer, welcher auch als Dichtung fungiert, und (D) eine Querschnittsansicht der in (B) gezeigten Ausführungsform mit einem an der unteren Oberflächenseite angeordneten Deckel (7).

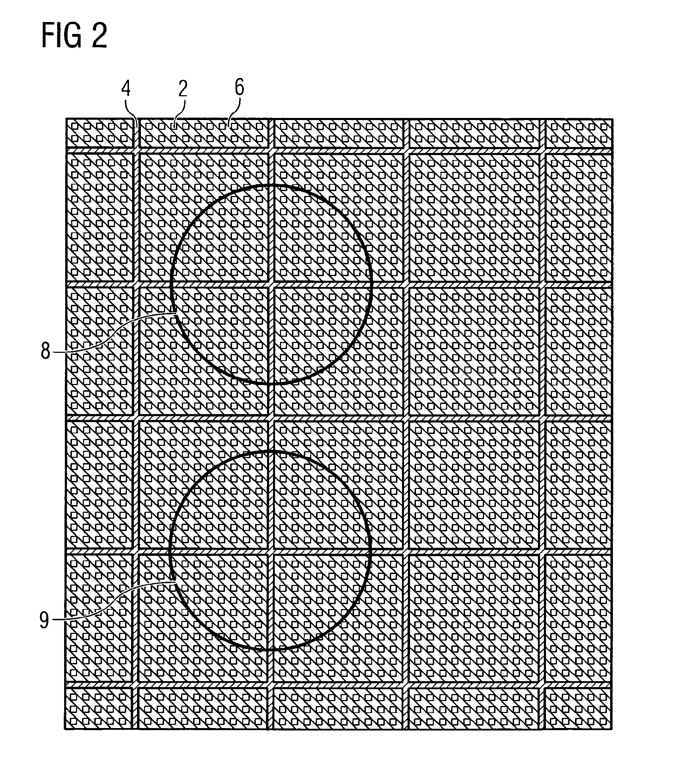

Fig. 2 zeigt eine schematische Draufsicht einer Ausführungsform einer erfindungsgemäßen Vorrichtung auf Basis von partiell oxidiertem makroporösen Silizium, worin (2) eine Pore, (4) eine Porenwand mit Siliziumkern, (6) eine Porenwand aus SiO₂, (8) einen ersten Bereich des Trägermaterials, in dem ein vorbestimmter Teil eines zur Amplifikationsreaktion befähigten Reaktionsgemischs bereitgestellt wird, und (9) einen zweiten Bereich des Trägermaterials, in dem ein vorbestimmter Teil eines Reaktionsgemischs bereitgestellt wird, der sich in mindestens einer Verbindung von dem im ersten Bereich vorgesehenen Teils des zur Amplifikationsreaktion befähigten Reaktionsgemischs unterscheidet, darstellen.

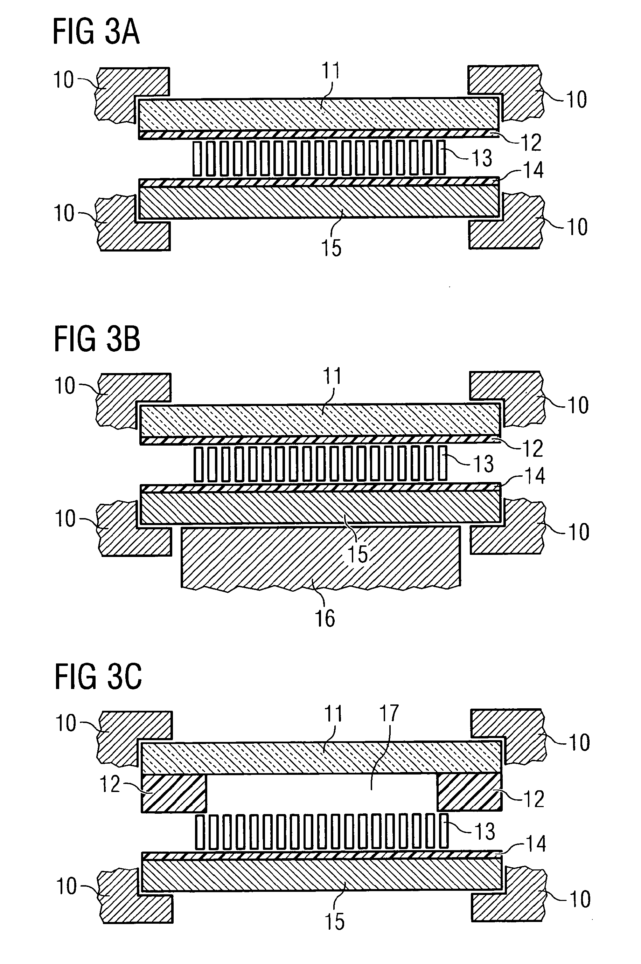

Fig. 3 zeigt eine Querschnittsansicht (A) einer Ausführungsform einer Durchführung der Amplifikationsreaktion, worin (10) eine Halterung mit geringer Wärmekapazität (z.B. aus Kunststoff), (11) ein transparentes Fenster (z.B. aus Glas), (12) eine chemisch inerte Dichtung von 1 bis 200 *µ*m (z. B. ein Silikon-Elastomer z.B. aus Polydimethylsiloxan), (13) eine erfindungsgemäße Vorrichtung, (14) eine chemisch inerte Dichtung von 1 bis 200 µm (z. B. ein Silikon-Elastomer z.B. aus Polydimethylsiloxan), (15) eine reflektierende, nicht lichtstreuende, chemisch inerte Unterlage mit hoher Wärmeleitfähigkeit (z.B. Silizium) ist, wobei die Halterung das Fenster und die Unterlage zusammendrückt, um eine Abdichtung zu erreichen; (B) eine Querschnittsansicht der in (A) gezeigten Ausführungsform mit einem Heiz- und/oder Kühlblock (16) und (C) eine Querschnittsansicht der in (A) gezeigten Ausführungsform mit einer Reaktionskammer (17) über der erfindungsgemäßen Vorrichtung darstellen.

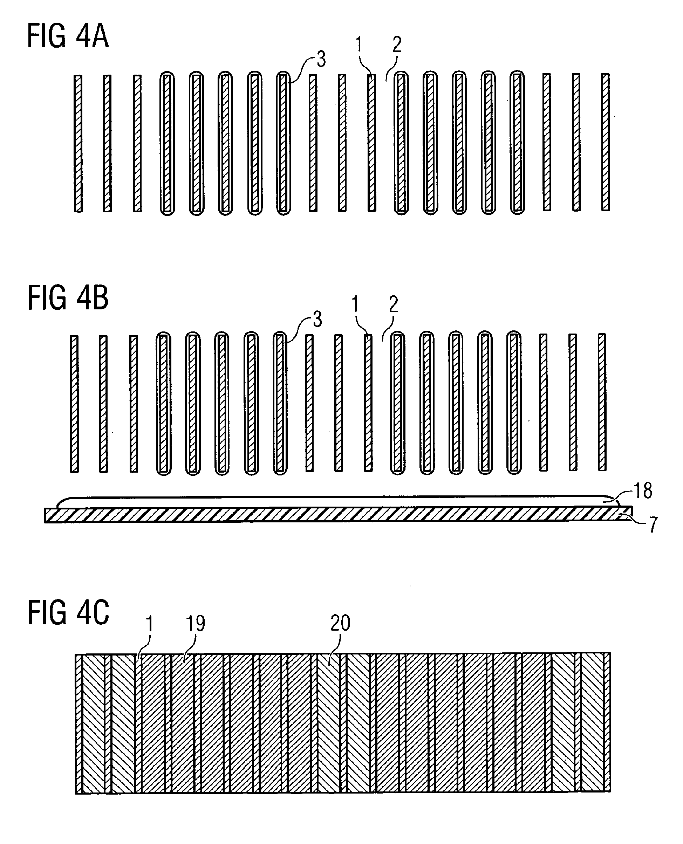

Fig. 4 zeigt eine Querschnittsansicht verschiedener Stadien des Hinzufügens einer Probe über das gesamte Trägermaterial, wobei (A) eine Querschnittsansicht einer Ausführungsform einer erfindungsgemäßen Vorrichtung, worin (1) eine Porenwand, (2) eine Pore und (3) eingetrocknete Verbindungen, den vorbestimmten Teil eines zur Amplifikationsreaktion befähigten Reaktionsgemischs bildend, darstellen; (B) die Querschnittsansicht einer Ausführungsform einer erfindungsgemäßen Vorrichtung und einen Deckel (7) mit einem auf der Oberflächenseite aufgebrachten Probenmaterial (18) und (C) die Querschnittsansicht einer Ausführungsform einer erfindungsgemäßen Vorrichtung nach dem Hinzufügen der Probe zeigt, wobei (19) eine Pore ist, die mit einem zur Amplifikation befähigten Reaktionsgemisch gefüllt ist und die in einem auf der Trägermaterialoberfläche definierten Reaktionsbereich liegt, und (20) eine Pore ist, die lediglich den zur Komplettierung für eine Amplifikationsreaktion erforderlichen Teil des Reaktionsgemischs enthält, so daß in einer solchen Pore keine Amplifikation stattfindet, und die zwischen den auf der Trägermaterialoberfläche definierten Reaktionsbereichen liegt, darstellen.

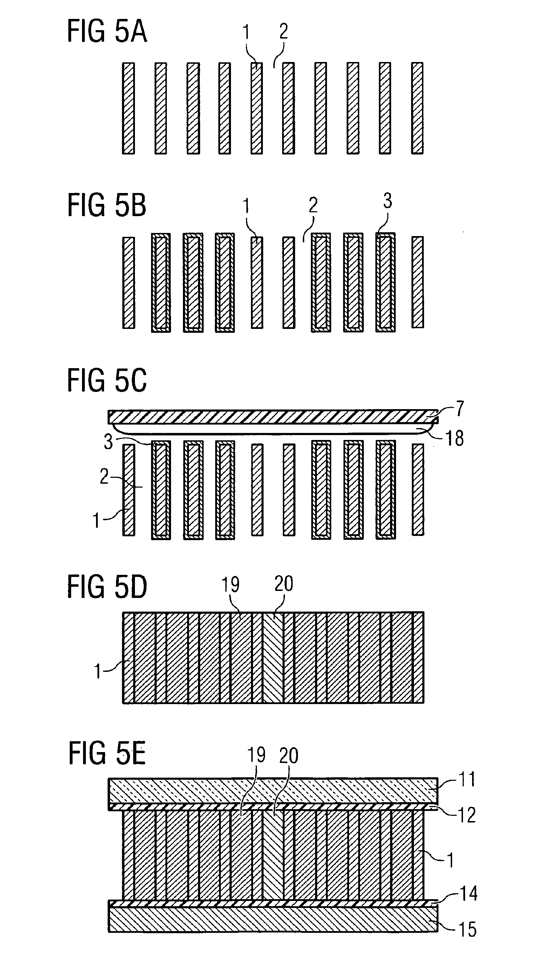

Fig. 5 zeigt eine Querschnittsansicht einer Ausführungsform des Bereitstellens des zur Amplifikationsreaktion befähigten Reaktionsgemischs auf dem Trägermaterial, wobei (A) eine Querschnittsansicht einer Ausführungsform einer erfindungsgemäßen Vorrichtung, worin (1) eine Porenwand, (2) eine Pore darstellen; (B) die Querschnittsansicht einer Ausführungsform einer erfindungsgemäßen Vorrichtung nach dem Bereitstellen eines vorbestimmten Teils eines zur Amplifikationsreaktion befähigten Reaktionsgemischs in mindestens einem Bereich des Trägermaterials, derart daß der Teil des Reaktionsgemischs nicht-kovalent an die Porenwand des Trägermaterials gebunden wird, so daß dadurch gleichzeitig ein Reaktionsbereich auf der Trägermaterialoberfläche definiert wird, wobei (3) eingetrocknete Verbindungen darstellt; (C) die Querschnittsansicht einer Ausführungsform einer erfindungsgemäßen Vorrichtung und einen Deckel (7) mit einem auf der Oberflächenseite aufgebrachten Probenmaterial (18) und (D) die Querschnittsansicht einer Ausführungsform einer erfindungsgemäßen Vorrichtung nach dem Hinzufügen der Probe zeigt, wobei (19) eine Pore ist, die mit einem zur Amplifikation befähigten Reaktionsgemisch gefüllt ist und die in einem auf der Trägermaterialoberfläche definierten Reaktionsbereich liegt, und (20) eine Pore ist, die lediglich den zur Komplettierung für eine Amplifikationsreaktion erforderlichen Teil des Reaktionsgemischs enthält, so daß in einer solchen Pore keine Amplifikation stattfindet, und die zwischen den auf der Trägermaterialoberfläche definierten Reaktionsbereichen liegt; (E) die Querschnittsansicht einer Ausführungsform einer Durchführung der Amplifikationsreaktion, worin (11) ein transparentes Fenster (z.B. aus Glas), (12) eine chemisch inerte Dichtung von 1 bis 200 *µ*m (z. B. ein Silikon-Elastomer z.B. aus Polydimethylsiloxan), (14) eine chemisch inerte Dichtung von 1 bis 200 *µ*m (z. B. ein Silikon-Elastomer z.B. aus Polydimethylsiloxan) und (15) eine reflektierende, nicht lichtstreuende, chemisch inerte Unterlage mit hoher Wärmeleitfähigkeit (z.B. Silizium) ist, darstellen.

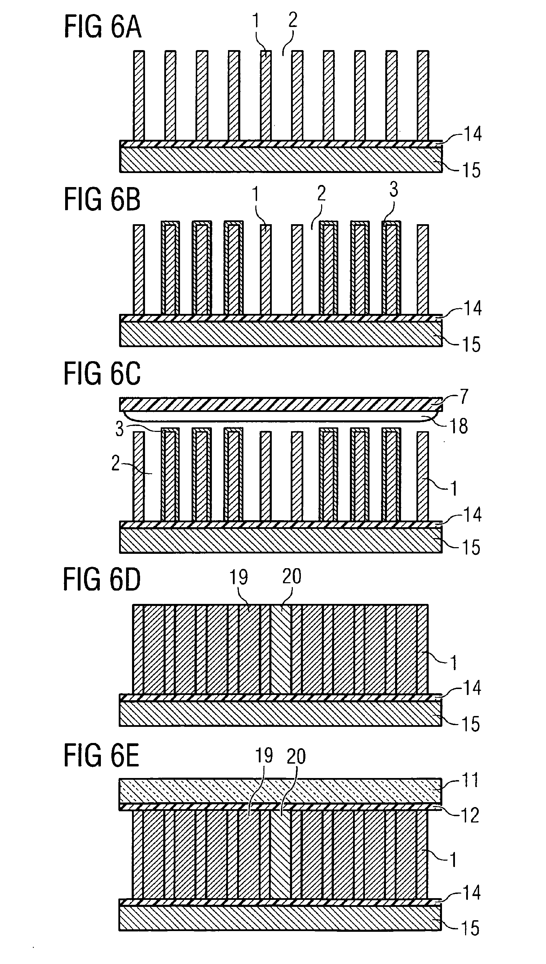

Fig. 6 zeigt eine Querschnittsansicht einer weiteren bevorzugten Ausführungsform des Bereitstellens des zur Amplifikationsreaktion befähigten Reaktionsgemischs auf dem Trägermaterial, wobei (A) eine Querschnittsansicht einer bevorzugten Ausführungsform einer erfindungsgemäßen Vorrichtung, worin (1) eine Porenwand, (2) eine Pore, (14) eine chemisch inerte Dichtung von 1 bis 200 *µ*m (z. B. ein Silikon-Elastomer z.B. aus Polydimethylsiloxan) und (15) eine reflektierende, nicht lichtstreuende, chemisch inerte Unterlage mit hoher Wärmeleitfähigkeit (z.B. Silizium) ist, darstellen; (B) die Querschnittsansicht einer Ausführungsform einer erfindungsgemäßen Vorrichtung nach dem Bereitstellen eines vorbestimmten Teils eines zur Amplifikationsreaktion befähigten Reaktionsgemischs in mindestens einem Bereich des Trägermaterials, derart daß der Teil des Reaktionsgemischs nicht-kovalent an die Porenwand des Trägermaterials gebunden wird, so daß dadurch gleichzeitig ein Reaktionsbereich auf der Trägermaterialoberfläche definiert wird, wobei (3) eingetrocknete Verbindungen darstellt; (C) die Querschnittsansicht einer Ausführungsform einer erfindungsgemäßen Vorrichtung und einen Deckel (7) mit einem auf der Oberflächenseite aufgebrachten Probenmaterial (18) und (D) die Querschnittsansicht einer Ausführungsform einer erfindungsgemäßen Vorrichtung nach dem Hinzufügen der Probe zeigt, wobei (19) eine Pore ist, die mit einem zur Amplifikation befähigten Reaktionsgemisch gefüllt ist und die in einem auf der Trägermaterialoberfläche definierten Reaktionsbereich liegt, und (20) eine Pore ist, die lediglich den zur Komplettierung für eine Amplifikationsreaktion erforderlichen Teil des Reaktionsgemischs enthält, so daß in einer solchen Pore keine Amplifikation stattfindet, und die zwischen den auf der Trägermaterialoberfläche definierten Reaktionsbereichen liegt; (E) die Querschnittsansicht einer Ausführungsform einer Durchführung der Amplifikationsreaktion, worin (11) ein transparentes Fenster (z.B. aus Glas) und (12) eine chemisch inerte Dichtung von 1 bis 200 *µ*m (z. B. ein Silikon-Elastomer z.B. aus Polydimethylsiloxan) darstellen.

### Bezugszeichenliste

(1) Porenwand
(2) Pore
(3) eingetrocknete Verbindungen, den vorbestimmten Teil eines zur Amplifikationsreaktion befähigten Reaktionsgemischs bildend
(4) Siliziumkern einer Porenwand,
(5) SiO₂
(6) Porenwand aus SiO₂
(7) Deckel auf Basis von z.B. einem Elastomer
(8) erster Bereich des Trägermaterials, in dem ein vorbestimmter Teil eines zur Amplifikationsreaktion befähigten Reaktionsgemischs bereitgestellt wird,
(9) zweiter Bereich des Trägermaterials, in dem ein vorbestimmter Teil eines Reaktionsgemischs bereitgestellt wird, der sich in mindestens einer Verbindung oder durch die Konzentration mindestens einer Verbindung von dem im ersten Bereich vorgesehenen Teils des zur Amplifikationsreaktion befähigten Reaktionsgemischs unterscheidet
(10) Halterung
(11) Fenster
(12) Dichtung
(13) erfindungsgemäße Vorrichtung
(14) Dichtung
(15) Unterlage
(16) Heiz- und/oder Kühlblock
(17) Reaktionskammer
(18) Probenmaterial
(19) in einem auf der Trägermaterialoberfläche definierten Reaktionsbereich liegende Pore, gefüllt mit einem zur Amplifikation befähigten Reaktionsgemisch
(20) zwischen den auf der Trägermaterialoberfläche definierten Reaktionsbereichen liegende Pore, wobei die Pore lediglich den zur Komplettierung für eine Amplifikationsreaktion erforderlichen Teil des Reaktionsgemischs enthält

## Patentansprüche

1. Verfahren zur Amplifikation von Nukleinsäuren in Poren eines flächig ausgebildeten makroporösen Trägermaterials, das gegenüberliegend eine erste und zweite Oberfläche aufweist, wobei über den gesamten Oberflächenbereich verteilt eine Vielzahl von diskreten Poren mit einem Durchmesser im Bereich von 500 nm bis 100 µm, einem Aspektverhältnis von Porentiefe zu Porenöffnung von mindestens 10:1 und einer Porendichte von 10⁴ bis 10⁸/cm² angeordnet ist, umfassend
(a) das Bereitstellen von einem vorbestimmten Teil eines zur Amplifikationsreaktion befähigten Reaktionsgemischs in mindestens einem Bereich des Trägermaterials, der mindestens zwei Poren umfaßt, derart daß der Teil des Reaktionsgemischs nicht-kovalent an die Porenwand des Trägermaterials gebunden wird, so daß **dadurch** gleichzeitig ein Reaktionsbereich auf der Trägermaterialoberfläche definiert wird,
(b) das Hinzufügen einer Probe über das gesamte Trägermaterial, die den zur Komplettierung für eine Amplifikationsreaktion erforderlichen Teil des Reaktionsgemischs enthält,
(c) das Durchführen der Amplifikationsreaktion, und
(d) das Nachweisen von mindestens einem Amplifikationsprodukt.

2. Verfahren nach Anspruch 1, weiter umfassend das Bereitstellen von mindestens einem weiteren Teil eines zur Amplifikationsreaktion befähigten Reaktionsgemischs, dessen Zusammensetzung sich von der Zusammensetzung des Teils des Reaktionsgemischs in Schritt (a) durch mindestens eine Verbindung oder durch die Konzentration mindestens einer Verbindung unterscheidet, in mindestens einem zweiten Bereich des Trägermaterials, der mindestens zwei Poren umfaßt und sich von dem Bereich in Schritt (a) unterscheidet.

3. Verfahren nach Anspruch 1 oder 2, wobei die vorbestimmten Teile eines zur Amplifikationsreaktion befähigten Reaktionsgemischs durch Eintrocknen einer diese Teile enthaltenen wässrigen Lösung an die Porenwand des Trägermaterials gebunden worden sind.

4. Verfahren nach einem der Ansprüche 1 bis 3, wobei das makroporöse Trägermaterial makroporöses Silizium oder partiell oxidiertes makroporöses Silizium ist.

5. Vorrichtung, umfassend ein flächig ausgebildetes makroporöses Trägermaterial, das gegenüberliegend eine erste und zweite Oberfläche aufweist, wobei über den gesamten Oberflächenbereich verteilt eine Vielzahl von diskreten Poren mit einem Durchmesser im Bereich von 500 nm bis 100 µm, einem Aspektverhältnis von Porentiefe zu Porenöffnung von mindestens 10:1 und einer Porendichte von 10⁴ bis 10⁸/cm² angeordnet ist, wobei die Vorrichtung mindestens einen Bereich aufweist, in dem ein vorbestimmter Teil eines Reaktionsgemischs zur Durchführung einer Amplifikation von Nukleinsäuren in mindestens zwei Poren nicht-kovalent gebunden ist.

6. Vorrichtung nach Anspruch 5, wobei die Vorrichtung mindestens einen weiteren Bereich aufweist, in dem ein vorbestimmter Teil eines Reaktionsgemischs zur Durchführung einer Amplifikation von Nukleinsäuren, dessen Zusammensetzung sich von der Zusammensetzung des Teils des Reaktionsgemischs im ersten Bereich durch mindestens eine Verbindung unterscheidet, in mindestens zwei Poren nicht-kovalent gebunden ist.

7. Vorrichtung nach Anspruch 5 oder 6, wobei mindestens eine Oberflächenseite des Trägermaterials mit einem Deckel verschlossen ist.

8. Vorrichtung nach einem der Ansprüche 5 bis 7, wobei das makroporöse Trägermaterial makroporöses Silizium oder partiell oxidiertes makroporöses Silizium ist.

9. Vorrichtung nach einem der Ansprüche 5 bis 8, wobei die Poren einen Durchmesser im Bereich von 2 bis 20 µm aufweisen.

10. Vorrichtung nach einem der Ansprüche 5 bis 9, wobei das Trägermaterial eine Dicke zwischen 100 bis 5.000 µm aufweist.

11. Verwendung eines flächig ausgebildeten makroporösen Trägermaterials, das gegenüberliegend eine erste und zweite Oberfläche aufweist, wobei über den gesamten Oberflächenbereich verteilt eine Vielzahl von diskreten Poren mit einem Durchmesser im Bereich von 500 nm bis 100 µm, einem Aspektverhältnis von Porentiefe zu Porenöffnung von mindestens 10:1 und einer Porendichte von 10⁴ bis 10⁸/cm² angeordnet ist, für die Amplifikation von Nukleinsäuren.
